(19) **Europäisches Patentamt**
**European Patent Office**
**Office européen des brevets**

(11) **EP 4 085 838 B1**

(12) **EUROPEAN PATENT SPECIFICATION**

(45) Date of publication and mention
of the grant of the patent:
**09.07.2025 Bulletin 2025/28**

(21) Application number: **21195209.8**

(22) Date of filing: **07.09.2021**

(51) International Patent Classification (IPC):
*A61B 5/024* *(2006.01)*  *A61B 5/327* *(2021.01)*
*A61B 5/352* *(2021.01)*  *A61B 5/361* *(2021.01)*
*G06N 3/044* *(2023.01)*  *G06N 3/045* *(2023.01)*
*G06N 3/047* *(2023.01)*  *G06N 3/048* *(2023.01)*
*G06N 3/084* *(2023.01)*  *G06N 3/088* *(2023.01)*
*G16H 50/20* *(2018.01)*  *G16H 50/70* *(2018.01)*
*A61B 5/319* *(2021.01)*  *G16H 50/50* *(2018.01)*
*G06N 3/082* *(2023.01)*

(52) Cooperative Patent Classification (CPC):
**A61B 5/02405; A61B 5/319; A61B 5/327;**
**A61B 5/352; A61B 5/361; G06N 3/044;**
**G06N 3/045; G06N 3/047; G06N 3/048;**
**G06N 3/088; G16H 50/20; G16H 50/50;**
**G16H 50/70;** G06N 3/082; G06N 3/084

(54) **ENSEMBLE GENERATIVE ADVERSARIAL NETWORK BASED SIMULATION OF CARDIOVASCULAR DISEASE SPECIFIC BIOMEDICAL SIGNALS**

AUF EINEM GENERATIVEN KONTRADIKTORISCHEN NETZWERK-ENSEMBLE BASIERTE SIMULATION VON KARDIOVASKULÄREN KRANKHEITSSPEZIFISCHEN BIOMEDIZINISCHEN SIGNALEN

SIMULATION DE SIGNAUX BIOMÉDICAUX SPÉCIFIQUES D'UNE MALADIE CARDIOVASCULAIRE BASÉE SUR UN RÉSEAU CONTRADICTOIRE GÉNÉRATIF D'ENSEMBLE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **03.05.2021 IN 202121020228**

(43) Date of publication of application:
**09.11.2022 Bulletin 2022/45**

(73) Proprietor: **Tata Consultancy Services Limited**
**Maharashtra (IN)**

(72) Inventors:
• **BANERJEE, ROHAN**
**700160 Kolkata - West Bengal (IN)**
• **GHOSE, AVIK**
**700160 Kolkata - West Bengal (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
• **HATAMIAN FAEZEH NEJATI ET AL: "The Effect of Data Augmentation on Classification of Atrial Fibrillation in Short Single-Lead ECG Signals Using Deep Neural Networks", ICASSP 2020 - 2020 IEEE INTERNATIONAL CONFERENCE ON ACOUSTICS, SPEECH AND SIGNAL PROCESSING (ICASSP), IEEE, 4 May 2020 (2020-05-04), pages 1264 - 1268, XP033793453, DOI: 10.1109/ICASSP40776.2020.9053800**
• **TOMER GOLANY ET AL: "SimGANs: Simulator-Based Generative Adversarial Networks for ECG Synthesis to Improve Deep ECG Classification", ARXIV.ORG, CORNELL UNIVERSITY LIBRARY, 201 OLIN LIBRARY CORNELL UNIVERSITY ITHACA, NY 14853, 27 June 2020 (2020-06-27), XP081707912**
• **YANG HAIXU ET AL: "ProEGAN-MS: A Progressive Growing Generative Adversarial Networks for Electrocardiogram Generation", IEEE ACCESS, IEEE, USA, vol. 9, 30 March 2021 (2021-03-30), pages 52089 - 52100, XP011848883, DOI: 10.1109/ACCESS.2021.3069827**

**(Cont. next page)**

EP 4 085 838 B1

• EDELMANN JAN-CHRISTOPH ET AL: "An ECG simulator with a novel ECG profile for physiological signals", JOURNAL OF MEDICAL ENGINEERING & TECHNOLOGY, vol. 42, no. 7, 3 October 2018 (2018-10-03), GB, pages 501 - 509, XP055892356, ISSN: 0309-1902, DOI: 10.1080/ 03091902.2019.1576788

**Description**

CROSS-REFERENCE TO RELATED APPLICATIONS AND PRIORITY

The present application claims priority to Indian application no. 202121020228, filed on May 3, 2021.

TECHNICAL FIELD

The disclosure herein generally relates to the field of simulation of cardiovascular disease specific biomedical signals, and, more particularly, to systems and methods for simulation of cardiovascular disease specific biomedical signals using an ensemble Generative Adversarial Network (GAN).

BACKGROUND

Electrocardiogram (ECG) is a clinical test to record the electrophysiological activities of the heart. The recorded data is interpreted by doctors for non-invasive diagnosis of cardiovascular diseases. However, it is practically impossible to manually analyze the large volume of ECG data generated each day in a hospital. An automatic diagnosis from the digitally recorded ECG is possible using artificial intelligence, machine learning, and deep learning techniques. A supervised learning-based cardiac diagnosis algorithm typically requires a large volume of annotated data as a prerequisite to creating the training model. Recording of large-scale patient data is time consuming and often challenging due to privacy issues and associated risks in case of infectious diseases. This is addressed in data science by generating synthetic patient data.

Although normal ECG samples are substantially available in various open-access databases, the quantity of abnormal recordings corresponding to different heart diseases is often inadequate to train a machine learning or deep learning classifier. The existing methods for simulating artificial ECG signals are mostly based on physics driven mathematical model. However, they require too many assumptions, for instance, parameters of the heart, thereby making them challenging to simulate on a large scale. On the other hand, deep learning-based approaches are also popular in literature. But these are pure data driven approach and hence, do not have physiological interpretation, for want of real data. Document Faezeh Nejati Hatamian et al., in "The Effect of Data Augmentation on Classification of Atrial Fibrillation in Short Single-Lead ECG Signals Using Deep Neural Networks″ discloses that: Cardiovascular diseases are the most common cause of mortality worldwide. Detection of atrial fibrillation (AF) in the asymptomatic stage can help prevent strokes. It also improves clinical decision making through the delivery of suitable treatment such as, anticoagulant therapy, in a timely manner. The clinical significance of such early detection of AF in electrocardiogram (ECG)signals has inspired numerous studies in recent years, of which many aim to solve this task by leveraging machine learning algorithms. ECG datasets containing AF samples, however, usually suffer from severe class imbalance, which if unaccounted for, affects the performance of classification algorithms. Data augmentation is a popular solution to tackle this problem. Hatamian et al., investigate the impact of various data augmentation algorithms, e.g., oversampling, Gaussian Mixture Models (GMMs) and Generative Adversarial Networks (GANs), on solving the class imbalance problem. These algorithms are quantitatively and qualitatively evaluated, compared and discussed in detail. The results show that deep learning-based AF signal classification methods benefit more from data augmentation using GANs and GMMs, than oversampling. Furthermore, the GAN results in circa 3% better AF classification accuracy in average while performing comparably to the GMM in terms of fl-score. Further, Document Tomer Golany et al., in "SimGANs: Simulator-Based Generative Adversarial Networks for ECG Synthesis to Improve Deep ECG Classification″ discloses that: Generating training examples for supervised tasks is a long sought after goal in AI. It studies the problem of heart signal electrocardiogram (ECG) synthesis for improved heartbeat classification. ECG synthesis is challenging: the generation of training examples for such biological physiological systems is not straightforward, due to their dynamic nature in which the various parts of the system interact in complex ways. However, an understanding of these dynamics has been developed for years in the form of mathematical process simulators. Tomer Golany et al., studies how to incorporate this knowledge into the generative process by leveraging a biological simulator for the task of ECG classification. Specifically, Tomer Golany et al., uses a system of ordinary differential equations (ODE) representing heart dynamics, and incorporate this ODE system into the optimization process of a generative adversarial network to create biologically plausible ECG training examples. Tomer Golany et al., performs empirical evaluation and show that heart simulation knowledge during the generation process improves ECG classification. Document HAIXU YANG et al., in "ProEGAN-MS: A Progressive Growing Generative Adversarial Network for Electrocardiogram Generation″ discloses that: Electrocardiogram (ECG) is a physiological signal widely used in monitoring heart health which is of great significance to the detection and diagnosis of heart diseases. Because abnormal heart rhythms are very rare, most ECG datasets have data imbalance problems. At present, many algorithms for ECG anomaly automatic recognition are affected by data imbalance. Conventional data augmentation methods are not suitable for the augmentation of the ECG signal, because the ECG signal is one-dimensional and their morphology has physiological significances. HAIXU YANG et al., proposes a ProGAN based ECG sample generation model, called ProEGAN-MS, to solve the problem of data imbalance. The model can stably generate realistic ECG samples. HAIXU YANG et al., evaluates the fidelity and diversity of the data generated by the model and compare the data distribution of the original and generated data. In addition, in order to show the diversity of the generated ECG data more intuitively, HAIXU YANG et al., manually checked the diversity and calculate the statistics of the data. The results show that compared with other ECG augmentation methods based on GANs, the ECG data generated by this model has higher fidelity and diversity, and the distribution of generated samples is closer to the distribution of original data. Finally, HAIXU YANG et al., established neural network models for arrhythmia classification, and used them to evaluate the improvement of the classification model performance by ProEGAN-MS.

SUMMARY

Embodiments of the present invention present technological improvements as solutions to one or more of the above-mentioned technical problems recognized by the inventors in conventional systems.

In an aspect, there is provided a processor implemented method as defined in appended independent claim 1.

In another aspect, there is provided a system as defined in appended independent claim 4.

In yet another aspect, there is provided a computer program product as defined in appended independent claim 7.

Favourable embodiments are set out in the dependent claims.

It is to be understood the following detailed description is exemplary and explanatory only and is not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG.1 illustrates an exemplary block diagram of a system for simulation of cardiovascular disease specific biomedical signals using an ensemble Generative Adversarial Network (GAN), in accordance with some embodiments of the present disclosure.

FIG.2 illustrates an exemplary architecture of the ensemble GAN for simulation of cardiovascular disease specific biomedical signals, in accordance with some embodiments of the present disclosure.

FIG.3A and FIG.3B illustrate Electrocardiogram (ECG) patterns of a normal subject and an Atrial Fibrillation (AF) patient, as known in the art.

FIG.4 illustrates an exemplary flow diagram of a computer implemented method for simulation of cardiovascular disease specific biomedical signals using the ensemble GAN, in accordance with some embodiments of the present disclosure.

FIG.5 illustrates sample ECG waveforms generated, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Artificial intelligence (AI), machine learning and deep learning techniques are used in healthcare applications for various diagnosis and simulation activities. The success of such algorithms heavily depends upon the size and the diversity of data used for training. However, recording of large-scale patient data is often difficult and time consuming due to underlying security and privacy issues as well as risks involved in case of infectious diseases.

The existing methods for simulating artificial Electrocardiogram (ECG) signals are mostly based on physics driven mathematical model. However, they require too many assumptions, for instance parameters of the heart, making them challenging to simulate on a large scale. On the other hand, deep learning-based approaches are purely data driven and hence, do not have physiological interpretation.

The deep learning approaches try to simulate artificial data by learning the distribution from a real-world training dataset. The Generative Adversarial Network (GAN) is a popular example of such generative modeling which is extensively used for generating realistic images and time-series data. Zhu et al. proposed a GAN architecture using the MIT-BIH arrhythmia database in Scientific reports 2019. However, the utility of the generated ECG data was not qualitatively evaluated. The GAN proposed by Hatamian et al. in ICASSP 2020 generates the spectrogram of ECG corresponding to Atrial Fibrillation (AF), but not the waveforms. Abnormal ECG waveforms contain various anomalous patterns owing to their underlying conditions, making them difficult to simulate using pure statistical approaches

The present disclosure effectively combines both physiological domain knowledge and deep learning to artificially simulate realistic cardiovascular disease specific biomedical signals. Atrial fibrillation (AF), a common type of arrhythmia has been used as an exemplary use case in the description. Although the method and system provided in the present disclosure may be applied to any biomedical signal such as ECG, Photoplethysmogram (PPG) or Phonocardiogram (PCG), the description below is directed to a non-limited example of periodic time series data (signal) such as ECG. Accordingly, ECG signal and biomedical signals may be used interchangeably. So also, cardiovascular disease and AF may be used interchangeably. In the claimed invention, the simulated time series data is limited to ECG.

In the context of the present disclosure, the expressions 'time series data', 'signal' and 'waveform' may be used interchangeably. Accordingly, multiple cycles form a waveform.

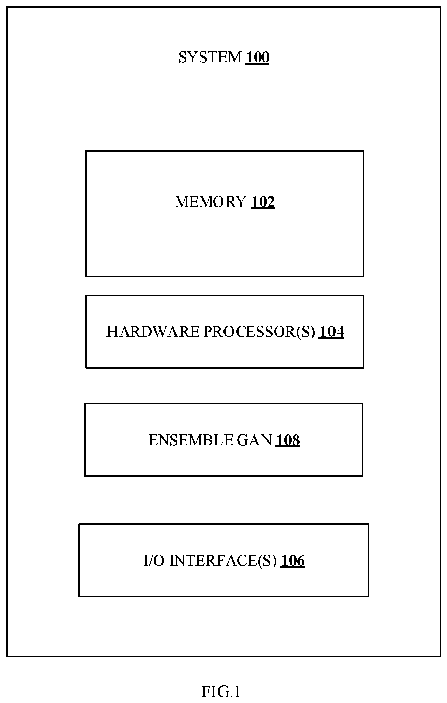

Referring now to the drawings, and more particularly to FIG. 1 through FIG.5, where similar reference characters denote corresponding features consistently throughout the figures, there are shown preferred embodiments and these embodiments are described in the context of the following exemplary system and/or method.

FIG.1 illustrates an exemplary block diagram of a system 100 for simulation of cardiovascular disease specific biomedical signals using an ensemble Generative Adversarial Network (GAN), in accordance with some embodiments of the present disclosure. In an embodiment, the system 100 includes one or more hardware processors 104, communication interface (s) or input/output (I/O) interface(s) 106, one or more data storage devices or memory 102 operatively coupled to the one or more hardware processors 104, and the ensemble GAN 108 described later in the description. The one or more hardware processors 104 can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, the system 100 can be implemented in a variety of computing systems, such as laptop computers, notebooks, hand-held devices, workstations, mainframe computers, servers, a network cloud and the like.

The communication interface (s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, one or more modules (not shown) of the system 100 can be stored in the memory 102.

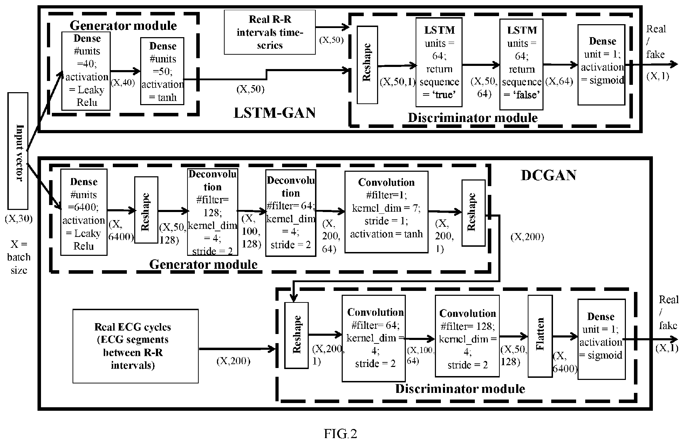

FIG.2 illustrates an exemplary architecture of the ensemble GAN for simulation of cardiovascular disease specific biomedical signals, in accordance with some embodiments of the present disclosure. In accordance with the present disclosure the ensemble GAN comprises a pair of GANs viz., (i) a Long Short-Term Memory GAN (LSTM-GAN) configured to generate a Heart Rate Variability (HRV) pattern associated with the cardiovascular disease condition and (ii) a Deep Convolutional GAN (DCGAN) configured to create a morphology of a representative cardiac cycle from the plurality of complete cardiac cycles, wherein each GAN in the pair of GANs includes a generator and a discriminator.

The generator $G$ takes an $N$-dimensional latent vector $z$ as input that follows a Gaussian distribution and maps it to a generated data as its output, $G(z)$. The discriminator $D$ outputs $D(G(z))$, a probability to predict whether the generated data is real, or fake based on a training set of real data, $x$. The generator and the discriminator reach a convergence state via a zero-sum game. The objective function of a GAN is expressed in terms of a min-max optimization process as in equation (1) below.

$$\min_{G} \max_{D} V(D, G) = E_{x \sim P(x)}[logD(x)] + E_{z \sim P(z)}[log(1 - D(G(z)))]$$

$$\rightarrow (1)$$

$D$ tries to maximize the probability to correctly classify real and fake data, and $G$ tries to minimize the probability that $D$ may predict its output as fake. Under an optimum state, distribution of the fake data becomes equivalent to the real data, and the discriminator classifies them at probability of 0.5.

An exemplary embodiment of the ensemble GAN of the present disclosure as shown in FIG. 2 is described herein below. A batch of 30-dimensional latent vectors randomly sampled from a standard normal distribution is fed to both generators as input during training. A set of annotated AF recordings are obtained from the PhysioNet Challenge 2017 database to form the real data.

The LSTM-GAN for generating R-R interval distances: Irregular HRV is a known clinical marker for AF. An LSTM is a deep learning architecture that has its internal memory for sequential modeling of time-series data in terms of a hidden vector. It can effectively learn the desired pattern from a very long sequence due to a unique cell structure, that enables to delete less important information from memory. The R-R interval distances extracted from an ECG data may be represented as a vector $rr_t$ of $k$ real numbers. Here, $rr_t = [rr_1, rr_2, ...... rr_k]$, where $rr_i = r_{i+1} - r_i$, and $r_i$ is the location of the $i^{th}R$ peak in the ECG signal on a time axis. The real R-R interval distances computed from the PhysioNet database for training the discriminator. The number of points in the vectors varies due to different lengths of the recordings in the database. Considering the median duration, length of an R-R intervals vector is set to 50 in the network. The shorter recordings are repeated and merged accordingly, whereas the longer recordings are broken into multiple partially overlapping segments to increase the instances of real data.

The generator and the discriminator are both designed using non-linear neural networks. Input to the generator is a set of real numbers having length of 30, randomly picked from a unit Gaussian distribution. The generator contains two dense layers (a set of fully connected neurons) that takes the input vector corresponding to the set of real numbers and maps to a vector which is equal to the number of neurons in the dense layers. The two dense layers of the generator have 40 and 50 neurons respectively, hence the output of the generator has a length of 50 which is the desired length of the generated R-R intervals by the architecture. Leaky Rectified Linear Unit (Leaky Relu) with negative slope coefficient =0.2 and hyperbolic tangent (tanh) functions are used for non-linear activation of the units in the two layers. The discriminator takes both the generator data and a reference R-R intervals time series as input and classifies whether the generated data is real or not. The inputs are required to be reshaped before applying them to the LSTM layers. The classifier in the discriminator contains a pair of LSTM layers (each having 64 units), that generate a hidden vector that maps the temporal relation of the input and then it is sent to a dense layer having a single neuron with sigmoid activation function for binary classification.

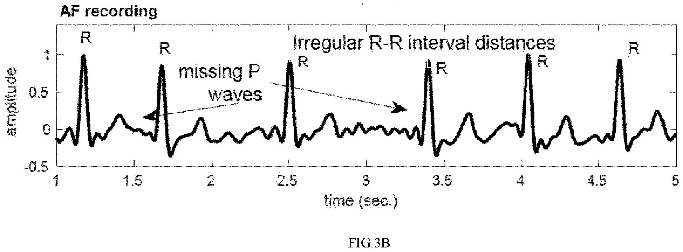

The DCGAN for generating signal morphology between two adjacent R peaks: Normal atrial activities of the heart become awry due to AF. This is reflected in the ECG morphology in terms of missing P waves or presence of abnormal fibrillatory waves before a QRS complex (Refer FIG.3B described below). The DCGAN structure to generate ECG cycles incorporating such unique morphology. As shown in equation (2) below, an ECG time-series, $ecg_t$ may be represented as a vector of m real numbers, containing a set of landmark points as the R peaks of known locations.

$$ecg_t = [ecg_1, ecg_2, .... ecg_{r_1}, ecg_{r_2}, ..... ecg_m$$

$$\rightarrow (2)$$

Here $r_i$ indicates the time location of the $i^{th}$ R peak in the ECG signal and $ecg_{r_i}$ is the corresponding amplitude. An ECG cycle is defined, in the present disclosure, as the segment between two adjacent R peaks. The $p^{th}$ cycle is extracted as given in equation (3) below.

$$cycle_p = [ecg_{\{r_p\}}, ecg_{\{r_{p+1}\}}, ecg_{\{r_{p+2}\}}, .... ecg_{\{r_{p+1}-1\}}]$$

$$\rightarrow (3)$$

In the DCGAN, the lower dimensional latent vector is converted to a desired space of realistic generated data based on a series of convolution and transposed-convolution operations in the discriminator and the generator through a set of filters (kernel). The real ECG cycles are extracted from the PhysioNet Challenge database. The signals are sampled at 300Hz. Since AF causes a heart rate faster than the normal range, length of an ECG cycle to be generated by the DCGAN model is fixed to 200 ($\approx$ 667 ms long, instantaneous heart rate = 90 bpm) in the architecture of the present disclosure. Duration of every real ECG cycle is modified accordingly using cubic spline interpolation technique before applying to the discriminator.

The generator takes the input vector of length 30 as input and first applies it to a dense layer having 6400 units to map it to a higher dimensional space. Subsequently, the output is applied to a pair of de-convolutional layers for extraction of relevant features. The output of the second deconvolutional layer is applied to another convolutional layer having a single filter and reshaped accordingly to get a required length, 150 of the generated cycle. The discriminator classifies whether the generated cycle is real or fake by comparing with a set of real cycles. After reshaping the classifier input for applying for convolution operation, the discriminator uses a pair of 1-Dimensional convolution layers with associated batch normalization and Leaky Relu activation layers. The convolution layers contain 64 and 128 filters respectively for relevant feature extraction. The output of the convolution operation is multidimensional. Hence, the output of the final convolutional layer is flattened to get a 1-Dimensional vector which is applied to a single neuron dense layer with sigmoid activation function for binary classification. To mitigate the chance of over-fitting, 30% dropout is applied to the convolutional layers. The generator is comprised of a dense layer and a pair of strided transposed-convolutional layers (also known as deconvolutional layers) having 128 and 64 filters (stride length = 2, kernel dimension = 4) with associated Leaky Relu layers to map the input latent vector to a higher dimensional space. There is a final convolutional layer, having a single filter of kernel dimension = 7 with a 'tanh' activation function to convert the feature-map to the desired shape of ECG cycle.

Training of the ensemble GAN: Separate mini-batches of real and fake data are used for training. The real and the fake samples are annotated as 1 and 0. The discriminator of each GAN aims to maximize the probability of correctly classifying an input as real or fake. The loss is expressed as $D_{loss}$ = log(D(x)) + log(1 - D(G(z))). These two terms are separately calculated on the mini-batches for real and generated fake data, providing a forward pass through the discriminator, and the gradients are calculated through a backward pass. For the generator, the loss term is $G_{loss}$ = log(D(G(z))). It tries to maximize log(D(G(z))), which is achieved by minimizing the term log(1 - D(G(z))). The loss is calculated based on the classification output of the generated data as predicted by the discriminator.

In order to ensure the ECG cycles generated by the DCGAN are close to real ECG morphology, the Mean Squared Error (MSE) between the real and the generated data is added to the generator loss function of the DCGAN as a penalty term to be minimized. Hence the total generator loss of the DCGAN is as shown in equation (4) below.

$$G_{loss_{tot}}^{DCGAN} = G_{loss}^{DCGAN} + \lambda * \frac{1}{n} \sum (Y_i - \hat{Y}_i)^2$$

$$\rightarrow (4)$$

$Y$ and $\hat{Y}$ are the real and generated data, $n$ is the batch size and the constant $\lambda$ controls the weight of the penalty term. A small value is set as $\lambda$ = 0.05, so that newer samples are generated keeping the morphology similar to real ECG cycles.

Adam optimizer with a learning rate of 0.0002 is used for the LSTM-GAN and the DCGAN. The mini-batch size is set as 64. The model weights are initialized from a normal distribution with zero mean and standard deviation of 0.02. Label smoothing is applied to modify the hard labels for real data slightly more or less than 1 and slightly more than 0 for fake data, where the variation for each label is done randomly. Additionally, some noise is introduced in the labels by randomly flipping the labels of a small fraction of real and fake data in each mini-batch. Both techniques have a regularization effect to avoid over-fit. The composite network is trained end to end up to 500 epochs applying the same set of latent vectors to the generator modules of the two GANs in every mini-batch.

Generating the complete ECG waveform: Once the training is done, the generator of the LSTM-GAN and the DCGAN can generate a vector of R-R interval distances of length 50 and an ECG cycle of length 200 from an input latent vector. The complete ECG waveform is created by modifying the length of the generated ECG cycle according to the R-R interval distances using cubic spline interpolation and merging them on time axis. The signal is applied to a 4th order Butterworth bandpass filter, having cut-off frequencies of 0.5 Hz and 20 Hz to remove the noise components.

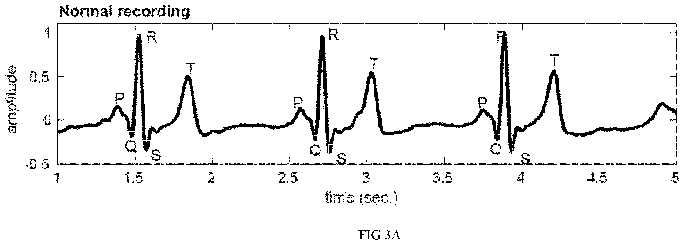

FIG.3A and FIG.3B illustrate ECG patterns of a normal subject and an AF patient, as known in the art. As seen in FIG.3A and FIG.3B, a normal ECG cycle has three major components, a P wave, a QRS complex containing an R peak, and the T wave. AF is a type of arrhythmia that affects the atrial activities of the heart. Firstly, the P waves are either absent or are replaced by fibrillatory waves. Secondly, the irregular Heart Rate Variability (HRV) due to AF causes large variation in successive R-R interval distances. These two clinical markers of AF are utilized in the present disclosure for synthesis of ECG signals.

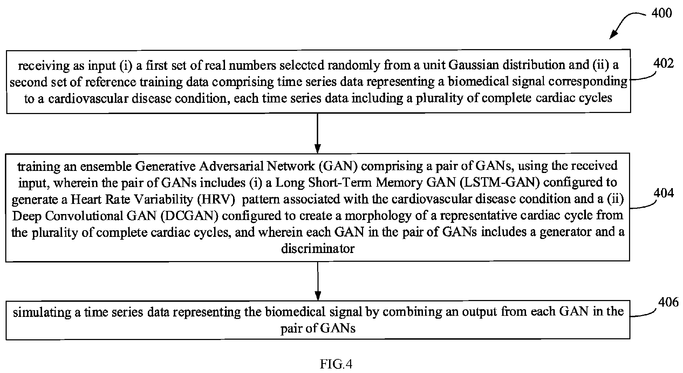

FIG.4 illustrates an exemplary flow diagram of a computer implemented method 400 for simulation of cardiovascular disease specific biomedical signals using the ensemble GAN, in accordance with some embodiments of the present disclosure. In an embodiment, the system 100 includes one or more data storage devices or memory 102 operatively coupled to the one or more hardware processors 104 and is configured to store instructions configured for execution of steps of the method 400 by the one or more hardware processors 104. The steps of the method 400 will now be explained in detail with reference to the components of the system 100 of FIG.1 and the exemplary architecture of FIG.2. Although process steps, method steps, techniques or the like may be described in a sequential order, such processes, methods and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

In an embodiment of the present disclosure, the one or more hardware processors 104, are configured to receive as input, at step 402, (i) a first set of real numbers selected randomly from a unit Gaussian distribution and (ii) a second set of reference training data comprising time series data representing a biomedical signal corresponding to a cardiovascular disease condition, each time series data including a plurality of complete cardiac cycles. The reference training data has the signature of the cardiovascular disease condition.

In an embodiment, the one or more hardware processors 104, are configured to train, at step 404, the ensemble GAN (108 of FIG.1) explained with reference to FIG.2 above. In an embodiment, the step of training the ensemble GAN comprises, performing for each training epoch, the steps of: generating an R-R interval time series, by the generator of the LSTM-GAN, using the first set of real numbers by mapping the first set of real numbers to a time series and classifying the generated R-R interval time-series as belonging to the cardiovascular disease condition or not, by the discriminator of the LSTM-GAN based on R-R interval distances computed using the second set of reference training data; and generating the representative cardiac cycle specific to the cardiovascular disease condition, by the generator of the DCGAN, using the first set of real numbers and classifying the generated representative cardiac cycle as belonging to the cardiovascular disease condition or not, by the discriminator of the DCGAN based on the cardiac cycle computed using the second set of reference training data. In accordance with the present disclosure, the step of training culminates after a first predefined number of training epochs or when the generated R-R interval time series and the generated representative cardiac cycle is within a predefined threshold when compared with the second set of reference training data.

In an embodiment, the step 404 of training the ensemble GAN is made efficient by validating the output (simulated time series data) after every second predefined number of training epochs. The time series data representing the biomedical signal is simulated by combining an output from each GAN in the pair of GANs, repeatedly, after the second predefined number of training epochs, where in the second predefined number of training epochs is less than the first predefined number of training epochs. For instance, an internal periodic validation may be set after every 5 training epochs or every 10 training epochs and the step of training may be stopped if the simulated time series data is within the predefined threshold when compared with the second set of reference training data (saturation of classification result). This makes the model more reliable. Once the training is complete, the discriminator may be removed and only the generator may be retained.

Further, the one or more hardware processors 104, are configured to simulate, at step 406, a time series data representing the biomedical signal by combining an output from each GAN in the pair of GANs. In an embodiment, the step of simulating the time series data representing the biomedical signal comprises modifying length of the representative cardiac cycle generated by the DCGAN according to the R-R interval distances generated by the LSTMGAN using cubic spline interpolation and appending the representative cardiac cycles on a time axis.

EXPERIMENTAL RESULTS

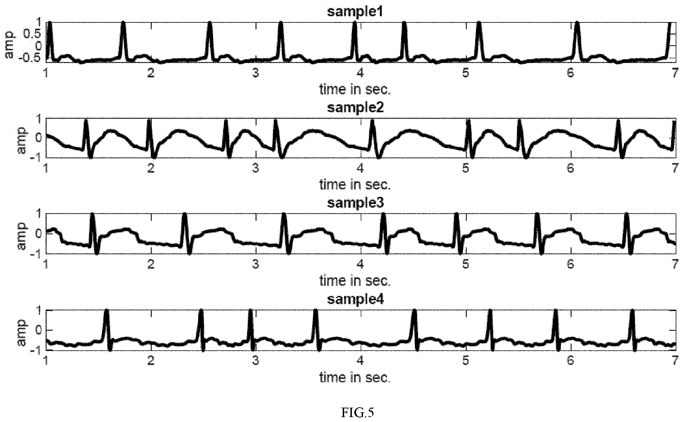

The architecture of the ensemble GAN of the present disclosure was implemented in Python™ 3.6 using TensorFlow 1.15 library. The training was performed on a computer with having Intel™ i7-7820X processor, 16 GB primary memory, and a GeForce™ GTX 1080 Ti graphics processing unit. FIG.5 illustrates sample ECG waveforms generated, in accordance with some embodiments of the present disclosure. Irregular HRV pattern, the primary marker for AF is visible in all of them. Absence of P wave before the QRS complex is found in the first two samples, whereas the remaining samples show traces of fibrillatory waves.

The efficacy of the system and method of the present disclosure in data augmentation to mitigate the class imbalance problem of a dataset is quantitatively evaluated on the PhysioNet Challenge 2017 database. This annotated ECG database contains 5154 normal, 771 AF, and 2557 other types of abnormal recordings. The annotated database is converted to a highly imbalanced database for binary classification by merging all types of non-AF recordings under a single class. The re-labelled database contains 7711 non-AF and 771 AF recordings. For evaluating the impact of data augmentation in classification performance, two state-of-the-art supervised learning-based AF detectors were selected which were validated on the original database.

The first algorithm is a classical machine learning approach by Datta et. al. (Computing, vol. 44, pp. 1, 2017), that trains a series of cascaded binary AdaBoost classifiers using more than 150 hand-crafted features, related to ECG morphology and short-term HRV. The second algorithm by Zihlmann et. al. (Computing, vol. 44, pp. 1, 2017), proposes two separate deep learning classifiers based on Convolutional Neural Network Architecture (CNN) and CRNN [combination of convolutional neural network (CNN) and recurrent neural network (RNN)], taking the 2-D spectrogram of ECG as input. The algorithms are modified to binary classifiers for the experimental purpose. 80% of data from both classes is selected for training and the remaining portion for testing. The AF portion in the training set is used for data augmentation to balance the class ratio of AF to non-AF data. Subsequently, the AF classifiers are trained on the balanced training set and evaluated on the test set. Classification performance is reported in terms of sensitivity (Se) and specificity (Sp) of detecting AF as shown in equation (5) below.

$$Se = \frac{TP}{TP + FN}, Sp = \frac{TN}{TN + FP}$$

→ (5)

*TP, TN, FP* and *FN* indicate the true positive, true negative, false positive and false negative respectively.

Table 1 below shows the performance improvement achieved by the first machine learning based algorithm, when the training is done incorporating the ensemble GAN architecture for data augmentation. A classifier trained on a highly imbalanced database is expected to be biased towards the majority class. A significant number of AF recordings are misidentified when the classifier is trained on the original dataset, resulting in high specificity and low sensitivity. Synthetic Minority Oversampling Technique (SMOTE) [Chawla et al., in Journal of artificial intelligence research, vol16, pp.321-357, 2002] and Adaptive Synthetic (ADASYN) [He et al., in IEEE, 2008, PP.1322-1328] are popularly used in machine learning for data augmentation. They simulate new data-points based on local information from the hand-crafted feature values computed from the AF-specific ECG signals. Although the sensitivity of AF improves due to them, there is a negative impact on specificity. The ensemble GAN, of the present disclosure generates newer ECG waveforms via learning the original class distribution. Hence, the features computed from the generated waveforms are found more effective than SMOTE and ADASYN, which significantly improves the classifier sensitivity without affecting the specificity.

Table 1: Quantitative analysis of the machine learning based AF classifier by Datta et. al., applying various data augmentation techniques to improve the class imbalance of the training set in the PhysioNet Challenge database.

| Augmentation (not used) | | Augmentation (SMOTE) | | Augmentation (ADASYN) | | Augmentation (ensemble GAN) | |
|---|---|---|---|---|---|---|---|
| *Se* | *Sp* | *Se* | *Sp* | *Se* | *Sp* | *Se* | *Sp* |
| 0.81 | 0.96 | 0.84 | 0.93 | 0.86 | 0.93 | 0.91 | 0.96 |

Table 2 below shows that the ensemble GAN based augmentation has a similar impact on the CNN and CRNN based AF classifiers by Zihlmann et. al. Here, the ensemble GAN architecture of the present disclosure is compared with two different approaches. The first approach is a popular trick in deep learning, where the AF classifier is trained on the original imbalanced dataset by assigning 10 times higher class weight to the minority class, which pays more attention to that class. In spite of a significant improvement in sensitivity, it shows a negative impact on specificity. The second approach is the GAN architecture by Hatamian et al. in IEEE 2020, pp. 1264-1268, that generates spectrogram of ECG without reconstructing the time-series. Unlike the second approach, the ensemble GAN, of the present disclosure is designed based on the clinical biomarkers of AF, showing the capability to generate realistic ECG to improve the diversity of the training set. Thus, it has the optimum impact on classifier performance.

Table 2: Quantitative analysis of the deep learning-based AF classifiers by Zihlmann et. al, applying various data augmentation techniques to improve the class imbalance of the training set.

| | Augmentation (not used) | | Augmentation (class weight) | | Augmentation (GAN by Hatamian et al.) | | Augmentation (ensemble GAN) | |
|---|---|---|---|---|---|---|---|---|
| | *Se* | *Sp* | *Se* | *Sp* | *Se* | *Sp* | *Se* | *Sp* |
| CNN | 0.79 | 0.99 | 0.89 | 0.91 | 0.83 | 0.98 | 0.92 | 0.99 |
| CRNN | 0.81 | 0.98 | 0.92 | 0.93 | 0.89 | 0.96 | 0.95 | 0.98 |

Thus, the present disclosure effectively combines two independent domain knowledges (i) physiological domain knowledge and (ii) deep learning to artificially simulate realistic cardiovascular disease specific biomedical signals. LSTM and CNN are the basic building blocks of LSTM-GAN and DCGAN respectively. Both LSTM and CNN are popular in deep learning. LSTMs are typically used for temporal analysis of a time-series to predict a next state, whereas, CNNs are mostly used for spatial analysis of images to extract its relevant features, which can be used for image classification, restoration and new image generation. Hence, CNN and LSTM are typically used in different domains. Biomedical signals such as ECG is a time-series, hence, to simulate it LSTM is an obvious choice for a person skilled in the art. However, it is found that LSTMs cannot always match ECG patterns. An ECG is complex in nature. Moreover, an abnormal ECG contains various anomalous patterns which is difficult to simulate using pure temporal analysis by an LSTM-GAN. Hence, DCGAN is introduced additionally on top, in the present disclosure which considers the morphology of an ECG cycle as an image to simulate a pattern base on spatial feature extraction by CNN. Thus, the ensemble GAN architecture including the LSTM-GAN and the DCGAN performs a detailed spatio-temporal modeling of biomedical signals such as the ECG signal, to regenerate more realistic artificial data and provides a technical advance to the ensemble GAN.

The written description describes the subject matter herein to enable any person skilled in the art to make and use the embodiments. The scope of the invention is defined by the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more hardware processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (400) comprising the steps of:

   receiving as input, via one or more hardware processors, (i) a first set of real numbers selected randomly from a unit Gaussian distribution and (ii) a second set of reference training data comprising time series data representing a biomedical signal corresponding to a cardiovascular disease condition, each time series data including a plurality of complete cardiac cycles (402);
   **characterized in that**:
   training an ensemble Generative Adversarial Network (GAN) comprising a pair of GANs, via the one or more hardware processors, using the received input, wherein the pair of GANs includes (i) a Long Short-Term Memory GAN (LSTM-GAN) configured to generate a Heart Rate Variability (HRV) pattern associated with the cardiovascular disease condition and (ii) a Deep Convolutional GAN (DCGAN) configured to create a morphology of a representative cardiac cycle from the plurality of complete cardiac cycles, wherein each GAN in the pair of GANs includes a generator and a discriminator (404), and wherein the step of training an ensemble GAN comprises performing, for each training epoch, the steps of:

generating an R-R interval time series, by the generator of the LSTM-GAN, using the first set of real numbers by mapping the first set of real numbers to a time series and classifying the generated R-R interval time-series as belonging to the cardiovascular disease condition or not, by the discriminator of the LSTM-GAN based on R-R interval distances computed using the second set of reference training data; and

generating the representative cardiac cycle specific to the cardiovascular disease condition, by the generator of the DCGAN, using the first set of real numbers and classifying the generated representative cardiac cycle as belonging to the cardiovascular disease condition or not, by the discriminator of the DCGAN based on the cardiac cycle computed using the second set of reference training data,

wherein the step of training culminates after a first predefined number of training epochs or when the generated R-R interval time series and the generated representative cardiac cycle is within a predefined threshold when compared with the second set of reference training data;

simulating, via the one or more hardware processors, a time series data representing the biomedical signal by combining an output from each GAN in the pair of GANs (406), wherein the simulated time series data representing the biomedical signal is an Electrocardiogram (ECG); and

training an atrial fibrillation (AF) classifier using the simulated time series data.

2. The processor implemented method of claim 1, further comprising periodically validating the step of training by simulating the time series data representing the biomedical signal by combining an output from each GAN in the pair of GANs, repeatedly, after every second predefined number of training epochs, where in the second predefined number of training epochs is less than the first predefined number of training epochs.

3. The processor implemented method of claim 2, wherein the step of simulating the time series data representing the biomedical signal comprises modifying length of the representative cardiac cycle generated by the DCGAN according to the R-R interval distances generated by the LSTMGAN using cubic spline interpolation and appending the representative cardiac cycles on a time axis.

4. A system (100) comprising:

one or more hardware processors (104);
one or more communication interfaces (106);
one or more data storage devices (102) operatively coupled to the one or more hardware processors and configured to store instructions configured for execution by the one or more hardware processors (104) to:

receive as input (i) a first set of real numbers selected randomly from a unit Gaussian distribution and (ii) a second set of reference training data comprising time series data representing a biomedical signal corresponding to a cardiovascular disease condition, each time series data including a plurality of complete cardiac cycles;
**characterized in that**:

train an ensemble Generative Adversarial Network (GAN) comprising a pair of GANs, using the received input, wherein the pair of GANs includes (i) a Long Short-Term Memory GAN (LSTM-GAN) configured to generate a Heart Rate Variability (HRV) pattern associated with the cardiovascular disease condition and a (ii) Deep Convolutional GAN (DCGAN) configured to create a morphology of a representative cardiac cycle from the plurality of complete cardiac cycles, wherein each GAN in the pair of GANs includes a generator and a discriminator, and wherein the one or more processors are configured to train the ensemble GAN by performing, for each training epoch, the steps of:

generating an R-R interval time series, by the generator of the LSTM-GAN, using the first set of real numbers by mapping the first set of real numbers to a time series and classifying the generated R-R interval time-series as belonging to the cardiovascular disease condition or not, by the discriminator of the LSTM-GAN based on R-R interval distances computed using the second set of reference training data; and

generating the representative cardiac cycle specific to the cardiovascular disease condition, by the generator of the DCGAN, using the first set of real numbers and classifying the generated representative cardiac cycle as belonging to the cardiovascular disease condition or not, by the discriminator of the DCGAN based on the cardiac cycle computed using the second set of reference training data,

wherein the step of training culminates after a first predefined number of training epochs or when the generated R-R interval time series and the generated representative cardiac cycle is within a predefined threshold when compared with the second set of reference training data;

simulate a time series data representing the biomedical signal by combining an output from each GAN in the pair of GANs, wherein the simulated time series data representing the biomedical signal is an Electrocardiogram (ECG); and
train an atrial fibrillation (AF) classifier using the simulated time series data.

5. The system of claim 4, wherein the one or more processors are configured to periodically validate the step of training by simulating the time series data representing the biomedical signal by combining an output from each GAN in the pair of GANs, repeatedly, after every second predefined number of training epochs, where in the second predefined number of training epochs is less than the first predefined number of training epochs.

6. The system of claim 5, wherein the one or more processors are configured to simulate the time series data representing the biomedical signal by modifying length of the representative cardiac cycle generated by the DCGAN according to the R-R interval distances generated by the LSTMGAN using cubic spline interpolation and appending the representative cardiac cycles on a time axis.

7. A computer program product comprising a non-transitory computer readable medium having a computer readable program embodied therein, wherein the computer readable program, when executed on a computing device, causes the computing device to:

receive as input, via one or more hardware processors, (i) a first set of real numbers selected randomly from a unit Gaussian distribution and (ii) a second set of reference training data comprising time series data representing a biomedical signal corresponding to a cardiovascular disease condition, each time series data including a plurality of complete cardiac cycles;
**characterized in that**:

training an ensemble Generative Adversarial Network (GAN) comprising a pair of GANs, via the one or more hardware processors, using the received input, wherein the pair of GANs includes (i) a Long Short-Term Memory GAN (LSTM-GAN) configured to generate a Heart Rate Variability (HRV) pattern associated with the cardiovascular disease condition and (ii) a Deep Convolutional GAN (DCGAN) configured to create a morphology of a representative cardiac cycle from the plurality of complete cardiac cycles, wherein each GAN in the pair of GANs includes a generator and a discriminator, and wherein the computer readable program further causes the computing device to perform the step of training an ensemble GAN by performing, for each training epoch, the steps of :

generating an R-R interval time series, by the generator of the LSTM-GAN, using the first set of real numbers by mapping the first set of real numbers to a time series and classifying the generated R-R interval time-series as belonging to the cardiovascular disease condition or not, by the discriminator of the LSTM-GAN based on R-R interval distances computed using the second set of reference training data; and
generating the representative cardiac cycle specific to the cardiovascular disease condition, by the generator of the DCGAN, using the first set of real numbers and classifying the generated representative cardiac cycle as belonging to the cardiovascular disease condition or not, by the discriminator of the DCGAN based on the cardiac cycle computed using the second set of reference training data,
wherein the step of training culminates after a first predefined number of training epochs or when the generated R-R interval time series and the generated representative cardiac cycle is within a predefined threshold when compared with the second set of reference training data;

simulating, via the one or more hardware processors, a time series data representing the biomedical signal by combining an output from each GAN in the pair of GANs, wherein the simulated time series data representing the biomedical signal is an Electrocardiogram (ECG); and
training an atrial fibrillation (AF) classifier using the simulated time series data.

8. The computer program product of claim 7, wherein the computer readable program further causes the computing device to periodically validate the step of training by simulating the time series data representing the biomedical signal by combining an output from each GAN in the pair of GANs, repeatedly, after every second predefined number of training epochs, wherein the second predefined number of training epochs is less than the first predefined number of training epochs.

9. The computer program product of claim 8, wherein the computer readable program further causes the computing device to simulate the time series data representing the biomedical signal by modifying length of the representative cardiac cycle generated by the DCGAN according to the R-R interval distances generated by the LSTM-GAN using cubic spline interpolation and appending the representative cardiac cycles on a time axis.

**Patentansprüche**

1. Prozessorimplementiertes Verfahren (400), das die folgenden Schritte umfasst:

Empfangen, als Eingabe, über einen oder mehrere Hardwareprozessoren, (i) eines ersten Satzes von reellen Zahlen, die zufällig aus einer Gaußschen Verteilung ausgewählt werden, und (ii) eines zweiten Satzes von Referenztrainingsdaten, die Zeitreihendaten umfassen, die ein biomedizinisches Signal darstellen, das einem Herz-Kreislauf-Krankheitszustand entspricht, wobei jede Zeitreihendaten eine Mehrzahl von vollständigen Herzzyklen beinhaltet (402);
**dadurch gekennzeichnet, dass**:

Trainieren eines Ensemble Generative Adversarial Network (GAN) umfassend ein Paar von GANs, über den einen oder die mehreren Hardwareprozessoren, unter Verwendung der empfangenen Eingabe, wobei das Paar von GANs Folgendes beinhaltet: (i) ein Long Short-Term Memory GAN (LSTM-GAN), das konfiguriert ist, um ein Herzfrequenzvariabilitätsmuster (HRV-Muster) zu erzeugen, das mit dem Herz-Kreislauf-Krankheitszustand assoziiert ist, und (ii) ein Deep Convolutional GAN (DCGAN), das konfiguriert ist, um eine Morphologie eines repräsentativen Herzzyklus aus der Mehrzahl von vollständigen Herzzyklen zu erzeugen, wobei jedes GAN in dem Paar von GANs einen Generator und einen Diskriminator (404) beinhaltet und wobei der Schritt des Trainierens eines Ensemble GAN das Durchführen, für jede Trainings-Epoche, folgende Schritte umfasst:

Erzeugen einer R-R-Intervallzeitreihe durch den Generator des LSTM-GAN unter Verwendung des ersten Satzes von reellen Zahlen durch Abbilden des ersten Satzes von reellen Zahlen auf eine Zeitreihe und Klassifizieren der erzeugten R-R-Intervallzeitreihe als zu dem Herz-Kreislauf-Krankheitszustand gehörend oder nicht durch den Diskriminator des LSTM-GAN basierend auf R-R-Intervallabständen, die unter Verwendung des zweiten Satzes von Referenztrainingsdaten berechnet werden; und
Erzeugen des repräsentativen Herzzyklus, der für den Herz-Kreislauf-Krankheitszustand spezifisch ist, durch den Generator des DCGAN unter Verwendung des ersten Satzes von reellen Zahlen und Klassifizieren des erzeugten repräsentativen Herzzyklus als zu dem Herz-Kreislauf-Krankheitszustand gehörend oder nicht durch den Diskriminator des DCGAN basierend auf dem Herzzyklus, der unter Verwendung des zweiten Satzes von Referenztrainingsdaten berechnet wird,
wobei der Schritt des Trainierens nach einer ersten vordefinierten Anzahl von Trainingsepochen oder wenn die erzeugte R-R-Intervallzeitreihe und der erzeugte repräsentative Herzzyklus innerhalb eines vordefinierten Schwellenwerts liegen, im Vergleich mit dem zweiten Satz von Referenztrainingsdaten gipfelt;

Simulieren, über den einen oder die mehreren Hardwareprozessoren, von Zeitreihendaten, die das biomedizinische Signal darstellen, durch Kombinieren einer Ausgabe von jedem GAN in dem Paar von GANs (406), wobei die simulierten Zeitreihendaten, die das biomedizinische Signal darstellen, ein Elektrokardiogramm (ECG) sind; und
Trainieren eines Vorhofflimmern(AF)-Klassifikators unter Verwendung der simulierten Zeitreihendaten.

2. Prozessorimplementiertes Verfahren nach Anspruch 1, das ferner das periodische Validieren des Schritts des Trainierens durch Simulieren der Zeitreihendaten, die das biomedizinische Signal darstellen, durch wiederholtes Kombinieren einer Ausgabe von jedem GAN in dem Paar von GANs nach jeder zweiten vordefinierten Anzahl von Trainingsepochen umfasst, wobei die zweite vordefinierte Anzahl von Trainingsepochen kleiner als die erste vordefinierte Anzahl von Trainingsepochen ist.

3. Prozessorimplementiertes Verfahren nach Anspruch 2, wobei der Schritt des Simulierens der Zeitreihendaten, die das biomedizinische Signal darstellen, das Modifizieren der Länge des repräsentativen Herzzyklus, der durch den DCGAN erzeugt wird, gemäß den R-R-Intervallabständen, die durch den LSTMGAN erzeugt werden, unter Verwendung von kubischer Spline-Interpolation und Anhängen der repräsentativen Herzzyklen auf einer Zeitachse umfasst.

4. System (100), das Folgendes umfasst:

einen oder mehrere Hardwareprozessoren (104);
eine oder mehrere Kommunikationsschnittstellen (106);
eine oder mehrere Datenspeichervorrichtungen (102), die betriebsfähig mit dem einen oder den mehreren Hardwareprozessoren gekoppelt und konfiguriert sind, um Anweisungen zu speichern, die zur Ausführung durch den einen oder die mehreren Hardwareprozessoren (104) konfiguriert sind, um:

als Eingabe (i) einen ersten Satz von reellen Zahlen, die zufällig aus einer Gaußschen Einheitsverteilung ausgewählt werden, und (ii) einen zweiten Satz von Referenztrainingsdaten zu empfangen, die Zeitreihendaten umfassen, die ein biomedizinisches Signal darstellen, das einem Herz-Kreislauf-Krankheitszustand entspricht, wobei jede Zeitreihendaten eine Mehrzahl von vollständigen Herzzyklen beinhaltet; **dadurch gekennzeichnet, dass**:

Trainieren eines Ensemble Generative Adversarial Network (GAN), das ein Paar von GANs umfasst, unter Verwendung der empfangenen Eingabe, wobei das Paar von GANs Folgendes beinhaltet: (i) ein Long Short-Term Memory GAN (LSTM-GAN), das konfiguriert ist, um ein Herzfrequenzvariabilitätsmuster (HRV-Muster) zu erzeugen, das mit dem Herz-Kreislauf-Krankheitszustand assoziiert ist, und (ii) ein Deep Convolutional GAN (DCGAN), das konfiguriert ist, um eine Morphologie eines repräsentativen Herzzyklus aus der Mehrzahl von vollständigen Herzzyklen zu erzeugen, wobei jedes GAN in dem Paar von GANs einen Generator und einen Diskriminator beinhaltet und wobei der eine oder die mehreren Prozessoren konfiguriert sind, um das Ensemble GAN durch Durchführen der folgenden Schritte für jede Trainingsepoche zu trainieren:

Erzeugen einer R-R-Intervallzeitreihe durch den Generator des LSTM-GAN unter Verwendung des ersten Satzes von reellen Zahlen durch Abbilden des ersten Satzes von reellen Zahlen auf eine Zeitreihe und Klassifizieren der erzeugten R-R-Intervallzeitreihe als zu dem Herz-Kreislauf-Krankheitszustand gehörend oder nicht durch den Diskriminator des LSTM-GAN basierend auf R-R-Intervallabständen, die unter Verwendung des zweiten Satzes von Referenztrainingsdaten berechnet werden; und
Erzeugen des repräsentativen Herzzyklus, der für den Herz-Kreislauf-Krankheitszustand spezifisch ist, durch den Generator des DCGAN unter Verwendung des ersten Satzes von reellen Zahlen und Klassifizieren des erzeugten repräsentativen Herzzyklus als zu dem Herz-Kreislauf-Krankheitszustand gehörend oder nicht durch den Diskriminator des DCGAN basierend auf dem Herzzyklus, der unter Verwendung des zweiten Satzes von Referenztrainingsdaten berechnet wird, wobei der Schritt des Trainierens nach einer ersten vordefinierten Anzahl von Trainingsepochen oder wenn die erzeugte R-R-Intervallzeitreihe und der erzeugte repräsentative Herzzyklus innerhalb eines vordefinierten Schwellenwerts liegen, im Vergleich mit dem zweiten Satz von Referenztrainingsdaten gipfelt;

Simulieren von Zeitreihendaten, die das biomedizinische Signal darstellen, durch Kombinieren einer Ausgabe von jedem GAN in dem Paar von GANs, wobei die simulierten Zeitreihendaten, die das biomedizinische Signal darstellen, ein Elektrokardiogramm (ECG) sind; und
Trainieren eines Vorhofflimmern(AF)-Klassifikators unter Verwendung der simulierten Zeitreihendaten.

5. System nach Anspruch 4, wobei der eine oder die mehreren Prozessoren konfiguriert sind, um den Schritt des Trainierens durch Simulieren der Zeitreihendaten, die das biomedizinische Signal darstellen, durch wiederholtes Kombinieren einer Ausgabe von jedem GAN in dem Paar von GANs nach jeder zweiten vordefinierten Anzahl von Trainingsepochen periodisch zu validieren, wobei die zweite vordefinierte Anzahl von Trainingsepochen kleiner als die erste vordefinierte Anzahl von Trainingsepochen ist.

6. System nach Anspruch 5, wobei der eine oder die mehreren Prozessoren konfiguriert sind, um die Zeitreihendaten, die das biomedizinische Signal darstellen, durch Modifizieren der Länge des repräsentativen Herzzyklus, der durch den DCGAN erzeugt wird, gemäß den R-R-Intervallabständen, die durch den LSTMGAN erzeugt werden, unter Verwendung von kubischer Spline-Interpolation und Anhängen der repräsentativen Herzzyklen auf einer Zeitachse zu simulieren.

**7.** Computerprogrammprodukt, das ein nichtflüchtiges computerlesbares Medium umfasst, in dem ein computerlesbares Programm verkörpert ist, wobei das computerlesbare Programm, wenn es auf einer Rechenvorrichtung ausgeführt wird, die Rechenvorrichtung zu Folgendem veranlasst:

Empfangen, als Eingabe, über einen oder mehrere Hardwareprozessoren, (i) eines ersten Satzes von reellen Zahlen, die zufällig aus einer Gaußschen Einheitsverteilung ausgewählt werden, und (ii) eines zweiten Satzes von Referenztrainingsdaten, die Zeitreihendaten umfassen, die ein biomedizinisches Signal darstellen, das einem Herz-Kreislauf-Krankheitszustand entspricht, wobei jede Zeitreihendaten eine Mehrzahl von vollständigen Herzzyklen beinhaltet;
**dadurch gekennzeichnet, dass**:

Trainieren eines Ensemble Generative Adversarial Network (GAN), das ein Paar von GANs umfasst, über den einen oder die mehreren Hardwareprozessoren unter Verwendung der empfangenen Eingabe, wobei das Paar von GANs Folgendes beinhaltet: (i) ein Long-Short-Term-Memory-GAN (LSTM-GAN), das konfiguriert ist, um ein Herzfrequenzvariabilitäts-(HRV-)Muster zu erzeugen, das mit dem Herz-Kreislauf-Krankheitszustand assoziiert ist, und (ii) ein Deep Convolutional GAN (DCGAN), das konfiguriert ist, um eine Morphologie eines repräsentativen Herzzyklus aus der Mehrzahl von vollständigen Herzzyklen zu erzeugen, wobei jedes GAN in dem Paar von GANs einen Generator und einen Diskriminator beinhaltet, und wobei das computerlesbare Programm ferner die Rechenvorrichtung veranlasst, den Schritt des Trainierens eines Ensemble GAN durch Durchführen, für jede Trainingsepoche, folgende Schritte durchzuführen:

Erzeugen einer R-R-Intervallzeitreihe durch den Generator des LSTM-GAN unter Verwendung des ersten Satzes von reellen Zahlen durch Abbilden des ersten Satzes von reellen Zahlen auf eine Zeitreihe und Klassifizieren der erzeugten R-R-Intervallzeitreihe als zu dem Herz-Kreislauf-Krankheitszustand gehörend oder nicht durch den Diskriminator des LSTM-GAN basierend auf R-R-Intervallabständen, die unter Verwendung des zweiten Satzes von Referenztrainingsdaten berechnet werden; und
Erzeugen des repräsentativen Herzzyklus, der für den Herz-Kreislauf-Krankheitszustand spezifisch ist, durch den Generator des DCGAN unter Verwendung des ersten Satzes von reellen Zahlen und Klassifizieren des erzeugten repräsentativen Herzzyklus als zu dem Herz-Kreislauf-Krankheitszustand gehörend oder nicht durch den Diskriminator des DCGAN basierend auf dem Herzzyklus, der unter Verwendung des zweiten Satzes von Referenztrainingsdaten berechnet wird,
wobei der Schritt des Trainierens nach einer ersten vordefinierten Anzahl von Trainingsepochen oder wenn die erzeugte R-R-Intervallzeitreihe und der erzeugte repräsentative Herzzyklus innerhalb eines vordefinierten Schwellenwerts liegen, im Vergleich mit dem zweiten Satz von Referenztrainingsdaten gipfelt;

Simulieren, über den einen oder die mehreren Hardwareprozessoren, von Zeitreihendaten, die das biomedizinische Signal darstellen, durch Kombinieren einer Ausgabe von jedem GAN in dem Paar von GANs, wobei die simulierten Zeitreihendaten, die das biomedizinische Signal darstellen, ein Elektrokardiogramm (ECG) sind; und
Trainieren eines Vorhofflimmern(AF)-Klassifikators unter Verwendung der simulierten Zeitreihendaten.

**8.** Computerprogrammprodukt nach Anspruch 7, wobei das computerlesbare Programm ferner die Rechenvorrichtung veranlasst, den Schritt des Trainierens durch Simulieren der Zeitreihendaten, die das biomedizinische Signal darstellen, durch wiederholtes Kombinieren einer Ausgabe von jedem GAN in dem Paar von GANs nach jeder zweiten vordefinierten Anzahl von Trainingsepochen periodisch zu validieren, wobei die zweite vordefinierte Anzahl von Trainingsepochen kleiner als die erste vordefinierte Anzahl von Trainingsepochen ist.

**9.** Computerprogrammprodukt nach Anspruch 8, wobei das computerlesbare Programm ferner die Rechenvorrichtung veranlasst, die Zeitreihendaten, die das biomedizinische Signal darstellen, durch Modifizieren der Länge des repräsentativen Herzzyklus, der durch den DCGAN erzeugt wird, gemäß den R-R-Intervallabständen, die durch den LSTM-GAN erzeugt werden, unter Verwendung von kubischer Spline-Interpolation und Anhängen der repräsentativen Herzzyklen auf einer Zeitachse zu simulieren.

**Revendications**

1. Procédé mis en oeuvre par processeur (400) comprenant les étapes consistant à

   recevoir en entrée, via un ou plusieurs processeurs matériels, (i) un premier ensemble de nombres réels sélectionnés aléatoirement à partir d'une distribution gaussienne unitaire et (ii) un second ensemble de données d'apprentissage de référence comprenant des données de série temporelle représentant un signal biomédical correspondant à un état de maladie cardiovasculaire, chaque donnée de série temporelle comprenant une pluralité de cycles cardiaques complets (402) ;
   **caractérisé en ce que** :

   l'apprentissage d'un réseau antagoniste génératif (GAN) d'ensemble comprenant une paire de GAN, via les un ou plusieurs processeurs matériels, en utilisant l'entrée reçue, dans lequel la paire de GAN comprend (i) un GAN à mémoire à long court terme (LSTM-GAN) configuré pour générer un motif de variabilité de fréquence cardiaque (HRV) associé à l'état de maladie cardiovasculaire et (ii) un GAN à convolution profonde (DCGAN) configuré pour créer une morphologie d'un cycle cardiaque représentatif à partir de la pluralité de cycles cardiaques complets, dans lequel chaque GAN dans la paire de GAN comprend un générateur et un discriminateur (404), et dans lequel l'étape d'apprentissage d'un GAN d'ensemble comprend l'exécution, pour chaque époque d'apprentissage, des étapes consistant à :

      générer une série temporelle d'intervalles R-R, par le générateur du LSTM-GAN, en utilisant le premier ensemble de nombres réels en mappant le premier ensemble de nombres réels sur une série temporelle et en classant la série temporelle d'intervalles R-R générée comme appartenant ou non à l'état de maladie cardiovasculaire, par le discriminateur du LSTM-GAN sur la base de distances d'intervalles R-R calculées en utilisant le second ensemble de données d'apprentissage de référence ; et
      générer le cycle cardiaque représentatif spécifique à l'état de maladie cardiovasculaire, par le générateur du DCGAN, en utilisant le premier ensemble de nombres réels et en classant le cycle cardiaque représentatif généré comme appartenant ou non à l'état de maladie cardiovasculaire, par le discriminateur du DCGAN sur la base du cycle cardiaque calculé en utilisant le second ensemble de données d'apprentissage de référence,
      dans lequel l'étape d'apprentissage culmine après un premier nombre prédéfini d'époques d'apprentissage ou lorsque la série temporelle d'intervalles R-R générée et le cycle cardiaque représentatif généré sont dans les limites d'un seuil prédéfini lorsqu'ils sont comparés au second ensemble de données d'apprentissage de référence ;

   simuler, via les un ou plusieurs processeurs matériels, des données de série temporelle représentant le signal biomédical en combinant une sortie de chaque GAN dans la paire de GAN (406), dans lequel les données de série temporelle simulées représentant le signal biomédical sont un électrocardiogramme (ECG) ; et
   entraîner un classificateur de fibrillation auriculaire (AF) en utilisant les données de série temporelle simulées.

2. Procédé mis en oeuvre par processeur selon la revendication 1, comprenant en outre la validation périodique de l'étape d'apprentissage en simulant les données de série temporelle représentant le signal biomédical en combinant une sortie de chaque GAN dans la paire de GAN, de manière répétée, après chaque second nombre prédéfini d'époques d'apprentissage, où le second nombre prédéfini d'époques d'apprentissage est inférieur au premier nombre prédéfini d'époques d'apprentissage.

3. Procédé mis en oeuvre par processeur selon la revendication 2, dans lequel l'étape de simulation des données de série temporelle représentant le signal biomédical comprend la modification de la longueur du cycle cardiaque représentatif généré par le DCGAN selon les distances d'intervalles R-R générées par le LSTM-GAN en utilisant une interpolation spline cubique et en ajoutant les cycles cardiaques représentatifs sur un axe temporel.

4. Système (100) comprenant :

   un ou plusieurs processeurs matériels (104) ;
   une ou plusieurs interfaces de communication (106) ;
   un ou plusieurs dispositifs de stockage de données (102) couplés de manière opérationnelle aux un ou plusieurs processeurs matériels et configurés pour stocker des instructions configurées pour une exécution par les un ou plusieurs processeurs matériels (104) pour :

recevoir en entrée (i) un premier ensemble de nombres réels sélectionnés aléatoirement à partir d'une distribution gaussienne unitaire et (ii) un second ensemble de données d'apprentissage de référence comprenant des données de série temporelle représentant un signal biomédical correspondant à un état de maladie cardiovasculaire, chaque donnée de série temporelle comprenant une pluralité de cycles cardiaques complets ;

**caractérisé en ce que** :

l'apprentissage d'un réseau antagoniste génératif (GAN) d'ensemble comprenant une paire de GAN, en utilisant l'entrée reçue, dans lequel la paire de GAN comprend (i) un GAN à mémoire à long court terme (LSTM-GAN) configuré pour générer un motif de variabilité de fréquence cardiaque (HRV) associé à l'état de maladie cardiovasculaire et (ii) un GAN à convolution profonde (DCGAN) configuré pour créer une morphologie d'un cycle cardiaque représentatif à partir de la pluralité de cycles cardiaques complets, dans lequel chaque GAN dans la paire de GAN comprend un générateur et un discriminateur, et dans lequel les un ou plusieurs processeurs sont configurés pour entraîner le GAN d'ensemble en exécutant, pour chaque époque d'apprentissage, les étapes consistant à:

générer une série temporelle d'intervalles R-R, par le générateur du LSTM-GAN, en utilisant le premier ensemble de nombres réels en mappant le premier ensemble de nombres réels sur une série temporelle et en classant la série temporelle d'intervalles R-R générée comme appartenant ou non à l'état de maladie cardiovasculaire, par le discriminateur du LSTM-GAN sur la base de distances d'intervalles R-R calculées en utilisant le second ensemble de données d'apprentissage de référence ; et

générer le cycle cardiaque représentatif spécifique à l'état de maladie cardiovasculaire, par le générateur du DCGAN, en utilisant le premier ensemble de nombres réels et en classant le cycle cardiaque représentatif généré comme appartenant ou non à l'état de maladie cardiovasculaire, par le discriminateur du DCGAN sur la base du cycle cardiaque calculé en utilisant le second ensemble de données d'apprentissage de référence,

dans lequel l'étape d'apprentissage culmine après un premier nombre prédéfini d'époques d'apprentissage ou lorsque la série temporelle d'intervalles R-R générée et le cycle cardiaque représentatif généré sont dans les limites d'un seuil prédéfini lorsqu'ils sont comparés au second ensemble de données d'apprentissage de référence ;

simuler des données de série temporelle représentant le signal biomédical en combinant une sortie de chaque GAN dans la paire de GAN, dans lequel les données de série temporelle simulées représentant le signal biomédical sont un électrocardiogramme (ECG) ; et

entraîner un classificateur de fibrillation auriculaire (AF) en utilisant les données de série temporelle simulées.

5. Système selon la revendication 4, dans lequel les un ou plusieurs processeurs sont configurés pour valider périodiquement l'étape d'apprentissage en simulant les données de série temporelle représentant le signal biomédical en combinant une sortie de chaque GAN dans la paire de GAN, de manière répétée, après chaque second nombre prédéfini d'époques d'apprentissage, où le second nombre prédéfini d'époques d'apprentissage est inférieur au premier nombre prédéfini d'époques d'apprentissage.

6. Système selon la revendication 5, dans lequel les un ou plusieurs processeurs sont configurés pour simuler les données de série temporelle représentant le signal biomédical en modifiant la longueur du cycle cardiaque représentatif généré par le DCGAN selon les distances d'intervalles R-R générées par le LSTMGAN en utilisant une interpolation spline cubique et en ajoutant les cycles cardiaques représentatifs sur un axe temporel.

7. Produit de programme informatique comprenant un support lisible par ordinateur non transitoire ayant un programme lisible par ordinateur incorporé dans celui-ci, dans lequel le programme lisible par ordinateur, lorsqu'il est exécuté sur un dispositif informatique, amène le dispositif informatique à :

recevoir en entrée, via un ou plusieurs processeurs matériels, (i) un premier ensemble de nombres réels sélectionnés aléatoirement à partir d'une distribution gaussienne unitaire et (ii) un second ensemble de données d'apprentissage de référence comprenant des données de série temporelle représentant un signal biomédical correspondant à un état de maladie cardiovasculaire, chaque donnée de série temporelle comprenant une pluralité de cycles cardiaques complets ;

**caractérisé en ce que** :

l'apprentissage d'un réseau antagoniste génératif (GAN) d'ensemble comprenant une paire de GAN, via les un ou plusieurs processeurs matériels, en utilisant l'entrée reçue, dans lequel la paire de GAN comprend (i) un GAN à mémoire à long court terme (LSTM-GAN) configuré pour générer un motif de variabilité de fréquence cardiaque (HRV) associé à l'état de maladie cardiovasculaire et (ii) un GAN à convolution profonde (DCGAN) configuré pour créer une morphologie d'un cycle cardiaque représentatif à partir de la pluralité de cycles cardiaques complets, dans lequel chaque GAN dans la paire de GAN comprend un générateur et un discriminateur, et dans lequel le programme lisible par ordinateur amène en outre le dispositif informatique à exécuter l'étape d'apprentissage d'un GAN d'ensemble en exécutant, pour chaque époque d'apprentissage, les étapes consistant à :

générer une série temporelle d'intervalles R-R, par le générateur du LSTM-GAN, en utilisant le premier ensemble de nombres réels en mappant le premier ensemble de nombres réels sur une série temporelle et en classant la série temporelle d'intervalles R-R générée comme appartenant ou non à l'état de maladie cardiovasculaire, par le discriminateur du LSTM-GAN sur la base de distances d'intervalles R-R calculées en utilisant le second ensemble de données d'apprentissage de référence ; et

générer le cycle cardiaque représentatif spécifique à l'état de maladie cardiovasculaire, par le générateur du DCGAN, en utilisant le premier ensemble de nombres réels et en classant le cycle cardiaque représentatif généré comme appartenant ou non à l'état de maladie cardiovasculaire, par le discriminateur du DCGAN sur la base du cycle cardiaque calculé en utilisant le second ensemble de données d'apprentissage de référence,

dans lequel l'étape d'apprentissage culmine après un premier nombre prédéfini d'époques d'apprentissage ou lorsque la série temporelle d'intervalles R-R générée et le cycle cardiaque représentatif généré sont dans les limites d'un seuil prédéfini lorsqu'ils sont comparés au second ensemble de données d'apprentissage de référence ;

simuler, via les un ou plusieurs processeurs matériels, des données de série temporelle représentant le signal biomédical en combinant une sortie de chaque GAN dans la paire de GAN, dans lequel les données de série temporelle simulées représentant le signal biomédical sont un électrocardiogramme (ECG); et

entraîner un classificateur de fibrillation auriculaire (AF) en utilisant les données de série temporelle simulées.

8. Produit de programme informatique selon la revendication 7, dans lequel le programme lisible par ordinateur amène en outre le dispositif informatique à valider périodiquement l'étape d'apprentissage en simulant les données de série temporelle représentant le signal biomédical en combinant une sortie de chaque GAN dans la paire de GAN, de manière répétée, après chaque second nombre prédéfini d'époques d'apprentissage, où le second nombre prédéfini d'époques d'apprentissage est inférieur au premier nombre prédéfini d'époques d'apprentissage.

9. Produit de programme informatique selon la revendication 8, dans lequel le programme lisible par ordinateur amène en outre le dispositif informatique à simuler les données de série temporelle représentant le signal biomédical en modifiant la longueur du cycle cardiaque représentatif généré par le DCGAN selon les distances d'intervalles R-R générées par le LSTM-GAN en utilisant une interpolation spline cubique et en ajoutant les cycles cardiaques représentatifs sur un axe temporel.

SYSTEM **100**

MEMORY **102**

HARDWARE PROCESSOR(S) **104**

ENSEMBLE GAN **108**

I/O INTERFACE(S) **106**

FIG.1

FIG.2

20

FIG.3A

FIG.3B

400

receiving as input (i) a first set of real numbers selected randomly from a unit Gaussian distribution and (ii) a second set of reference training data comprising time series data representing a biomedical signal corresponding to a cardiovascular disease condition, each time series data including a plurality of complete cardiac cycles

402

training an ensemble Generative Adversarial Network (GAN) comprising a pair of GANs, using the received input, wherein the pair of GANs includes (i) a Long Short-Term Memory GAN (LSTM-GAN) configured to generate a Heart Rate Variability (HRV) pattern associated with the cardiovascular disease condition and a (ii) Deep Convolutional GAN (DCGAN) configured to create a morphology of a representative cardiac cycle from the plurality of complete cardiac cycles, and wherein each GAN in the pair of GANs includes a generator and a discriminator

404

simulating a time series data representing the biomedical signal by combining an output from each GAN in the pair of GANs

406

FIG.4

FIG.5

24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202121020228

**Non-patent literature cited in the description**

- **FAEZEH NEJATI HATAMIAN et al.** *The Effect of Data Augmentation on Classification of Atrial Fibrillation in Short Single-Lead ECG Signals Using Deep Neural Networks*
- **TOMER GOLANY et al.** *SimGANs: Simulator-Based Generative Adversarial Networks for ECG Synthesis to Improve Deep ECG Classification*
- **HAIXU YANG et al.** *ProEGAN-MS: A Progressive Growing Generative Adversarial Network for Electrocardiogram Generation*
- **ZHU et al.** GAN architecture using the MIT-BIH arrhythmia database. *Scientific reports*, 2019
- **HATAMIAN et al.** *ICASSP*, 2020
- **DATTA**. *Computing*, 2017, vol. 44, 1
- **ZIHLMANN**. *Computing*, 2017, vol. 44, 1
- **CHAWLA et al.** *Journal of artificial intelligence research*, 2002, vol. 16, 321-357
- **HE et al.** *IEEE*, 2008, 1322-1328
- **HATAMIAN et al.** *IEEE*, 2020, 1264-1268